# EUROPEAN PATENT APPLICATION

(11) **EP 4 817 608 A1**
(43) Date of publication of application: **07.10.2026**
(21) Application number: 24896494.2
(22) Date of filing: 25.11.2024
(51) Int. Cl.: A61K 31/198, A61P 15/02, A61P 29/00, A61P 31/04, A61P 31/10

(54) **USE OF L-THEANINE IN PREPARATION OF FORMULATION FOR TREATING VAGINITIS**

(30) Priority: 29.11.2023 CN 202311615394
(71) Applicant: Honghua (Beijing) Technology Co., Ltd., Beijing 102299 (CN)
(72) Inventor: LIU, Cailing, Beijing 102299 (CN)
(74) Representative: dompatent
(86) International application number: PCT/CN2024/134376
(87) International publication number: WO 2025/113405

(57) **Abstract**

A use of L-theanine in the preparation of a formulation for treating vaginitis. The L-theanine can significantly reduce vaginal pH and improve the pathological score of the tissue, and is significantly effective for vaginitis.

## Description

### TECHNICAL FIELD

The present invention belongs to the field of novel pharmaceutical use, specifically relates to a use of L-theanine in the preparation of a formulation for treating vaginitis.

### BACKGROUND

The vaginal microbiota of healthy women is dominated by lactobacilli. The types of lactobacilli vary depending on the region and the individual, the main ones are L. crispatus, L. iners, and G. vaginalis. Among them, L. crispatus provides the strongest protection to the host, while L. iners and G. vaginalis offer relatively weaker protection. L. iners and G. vaginalis show low cholesterol-dependent cytolysin expression when coexisting with lactobacilli, but high expression when coexisting with other facultative and obligate anaerobes.

In a healthy state, the richness of vaginal microbial community is relatively low, with lactobacilli being the dominant species. Lactic acid is the primary vaginal acidifying substance, and protonated lactic acid can cause the vaginal pH to be less than 4.5 (average ~ 3.5), without inflammation.

In vaginitis, lactobacilli lose their dominant role, leading to an increase in the richness of the vaginal microbiota, with an increase in anaerobic and facultative anaerobic bacteria. Short - chain fatty acids (formic acid, acetic acid, propionic acid, butyric acid) are the main acidifying substances, but their acidifying ability is relatively weak, resulting in a vaginal pH > 4.5. Pathogenic bacteria decompose immature epithelial cells, producing biogenic amines, which cause inflammation and odor in the vagina. Common pathogenic bacteria include Gardnerella vaginalis, Group B Streptococcus, and Staphylococcus aureus, etc.

Antibiotic therapy is the standard treatment for vaginitis, and although it is highly effective, pathogens have developed varying degrees of resistance to antibiotics. In addition, people have tried to treat vaginitis by supplementing with exogenous lactobacilli and lactic acid, but both methods have some limitations: lactobacilli can only successfully colonize when the vaginal pH is <4.5, and exogenous lactic acid is not protonated lactic acid, resulting in a weak ability to maintain a pH <4.5 and inhibit bacterial growth. Therefore, lactobacilli and lactic acid therapy are often used sequentially with antibiotic therapy.

Prior art 1 (Xu Wei, Effect And Mechanism of L-Theanine on Short-Chain Fatty Acids Metabolism Related to Intestinal Mucosal Immune [D], Hunan Agricultural University, 2020) discloses that L-theanine can promote the growth of intestinal probiotics, but it can also promote the growth of intestinal pathogens, indicating that L-theanine is not a broad-spectrum antibacterial drug, but rather a microbial community regulator.

Prior art 2 (Ge Yan, Extraction, Isolation and Purification of Theanine from Summer - Autumn Green Tea and Its Influence on Intestinal Microecology [D]. Nanjing Agricultural University, 2014) discloses that L-theanine can increase the richness of intestinal microbiota and the content of short-chain fatty acids, suggesting that intravaginal administration of L-theanine may not be suitable for the treatment of vaginitis. As mentioned earlier, a healthy vagina should be dominated by lactobacilli with low microbiota richness. Increased microbiota richness and increased short-chain fatty acids are manifestations of vaginitis.

Prior art 2 also discloses that L-theanine can promote the secretion of intestinal lactic acid in humans, but the change in the number of lactic acid bacteria is not significant.

Prior art 3 (SAEED M. Influence of L-Theanine Dietary Supplementation on Growth Performance, Meat Quality, and Immune Response of Broiler Chickens [D], Northwest A&F University, 2018) discloses that L-theanine increases the Lactobacillus phylum, order, and genus in the ileum and colon of chickens, but does not disclose the bacterial strains, and the vaginal and intestinal vaginal strains are different.

Chinese patent application CN202310412887.4 discloses an L-theanine-containing fecal microbial solution. The Lactobacillus species in the L-theanine-containing fecal microbial solution are reuteri, Johnsonill, and Lactobacillus rhamnosus, which are not common vaginal colonizing bacteria.

Prior art 4 (Qu, Qing-Yun et al. "L-Theanine Modulates Intestine-Specific Immunity by Regulating the Differentiation of CD4+T Cells in Ovalbumin-Sensitized Mice." Journal of agricultural and food chemistry vol. 70, 47(2022):14851-14863. doi:10.1021/acs.jafc.2c06171) discloses that L-theanine reduces L. iners in a dosage-dependent manner , and L. iners is the most abundant lactobacilli in the vagina, which have a protective effect on the vagina.

Prior art 5 (France, Michael T et al. "Insight into the ecology of vaginal bacteria through integrative analyses of metagenomic and metatranscriptomic data." Genome biology vol. 23, 1 66. 1 Mar. 2022, doi:10.1186/s13059-022-02635-9) discloses that the transcriptional activity of L. crispatus and L. iners varies with the taxonomic composition of their community.

It can be seen that it is not known from the prior art whether L-theanine can promote the growth of vaginal lactobacilli *in vitro*, whether it can promote the growth of lactobacilli *in vivo*, and whether L-theanine can be used for the treatment of vaginitis. Therefore, the prior art has not disclosed the use of L-theanine in vaginitis.

### SUMMARY OF THE INVENTION

Therefore, the present invention conducted a pharmacodynamic study on L-theanine for vaginitis (bacterial vaginosis + aerobic vaginitis + candidal vaginitis and atrophic vaginitis models). The inventor unexpectedly discovered that L-theanine can significantly improve vaginitis symptoms, including disappearance of odor and restoration of normal vaginal discharge.

The present invention provides the use of L-theanine in the preparation of a formulation for treating vaginitis.

In a preferred embodiment of the use of the present invention, the vaginitis preferably comprises bacterial vaginitis, aerobic vaginitis, fungal vaginitis, atrophic vaginitis, and mixed vaginitis.

In a preferred embodiment of the use of the present invention, the administration route of L-theanine is preferably vaginal administration.

In a preferred embodiment of the use of the present invention, the effective therapeutic concentration of L-theanine for vaginal administration is preferably 0.1 w/w%~2.5 w/w%.

In a preferred embodiment of the use of the present invention, the effective therapeutic dosage of L-theanine for vaginal administration is preferably 10 mg~250 mg per day.

Experimental results show that the L-theanine of the present invention can significantly reduce vaginal pH, improve histopathological scores, and is significantly effective for vaginitis.

### DESCRIPTION OF THE DRAWINGS

Figure 1 shows the effect of L-theanine aqueous solution on vaginal bacterial colonization and cleanliness in a rat model of mixed vaginitis (X 100);
Figure 2 shows the effect of L-theanine aqueous solution on vaginal pathological changes (HE, X 200).

### DETAILED DESCRIPTION

The present invention will be further illustrated by the following examples, but it should be understood that the examples are for illustrative purposes only and not for limiting the invention.

### Example 1: Pharmacodynamic study of L-theanine aqueous solution in rats with mixed vaginitis

The formulations containing L-theanine proposed in the present invention can be in the form of solution, vaginal gel, vaginal suppository, vaginal tablet, or capsule. The preparation methods of these formulations are well known to those skilled in the art.

### Laboratory animals

Seventy SPF grade SD rats, female, weighing 193.7-254.4 g.

### Preparation of infectious bacterial solution

After three strains of Candida albicans (CMCC(F)98001), Group B Streptococcus (ATCC12386), and Gardnerella vaginalis (BNCC 337545) were revived, they were cultured and passaged in sterile nutrient broth, and a bacterial solution with a concentration of 2.0 × 10⁹ cells/ml was prepared. The three bacterial solutions were then mixed in a 1:1:1 ratio to prepare an infectious bacterial solution for later use.

### Establishment of animal models

Seventy SPF-grade female SD rats, weighing 193.7-254.4, were used. Ten rats were selected as a normal control group, while the remaining rats were anesthetized with isoflurane inhalation, and their bilateral ovaries were removed in a sterile operating table. After surgery, 200,000 units/rat of penicillin were injected intramuscularly for 3 consecutive days. Starting from the 4th day after surgery, estradiol benzoate was injected subcutaneously at a dosage of 10 mg/kg daily for four days. Then, hydrocortisone injection was administered subcutaneously at a dosage of 60 mg/kg every other day for three days to replicate the rat model of immunodeficiency. Two days later, 50 µL of infectious bacterial solution (1:1:1) was injected into the rat vagina. The rat's tail was lifted and held for approximately 2 minutes to prevent leakage. One day later, the same method was used for a second infection, and then estradiol benzoate and hydrocortisone injection were administered subcutaneously alternately once every other day. After the modeling was completed, the vulva of the rats was observed. If there was swelling, redness, and white secretion exudation, a small amount of secretion was applied to blood agar medium and cultured at 37°C. After 72 hours, colonies of varying sizes were observed on the medium, indicating the successful establishment of the model. Fifty rats with successful model establishment were randomly divided into a model control group, a compound metronidazole vaginal suppository group, and (0.5w/w%, 1.25w/w%, 2.5w/w%) L-theanine aqueous solution groups, with 10 rats in each group. Except for the normal control group and the model control group, which received 0.9% sodium chloride injection vaginally, the other groups received the medication vaginally, with an administration volume of 0.1g/rat, once daily for 14 consecutive days. Vaginal secretions were observed and photographed on D0 and D5, D10, and D14 after administration. Vaginal pH was tested weekly. After the last administration, vaginal smears and vaginal lavage fluid bacterial cultures were performed to calculate the negative conversion rate and colony count. Vaginal tissue pathology scores were also performed to evaluate pathological changes.

### Trial grouping and dosage

**Table 1. Trial grouping and dosage design**

| Groups | Times of administration (time/day) | Dosage (mL/rat) | Numbers of animal (rats) |
|---|---|---|---|
| Normal control group | -- | 0.1 | 10 |
| Model control group | -- | 0.1 | 10 |
| Compound metronidazole vaginal suppository group | 1 | 0.1g/rat | 10 |
| 0.1 w/w % L-theanine aqueous solution group | 1 | 0.1 | 10 |
| 0.5 w/w % L-theanine aqueous solution group | 1 | 0.1 | 10 |
| 2.5 w/w % L-theanine aqueous solution group | 1 | 0.1 | 10 |

### Detection indicators

**Vaginal smears:** after the last administration, vaginal secretions were collected from rats in each group using sterile cotton swabs for vaginal smear, followed by Wright staining and Gram staining. The smears were then applied to blood agar medium and incubated in a 37°C incubator. Colonies were observed visually after 24 hours. This was repeated twice, with a 12-hour interval between each observation. The absence of bacterial colonies in both observations indicated a negative conversion. The negative conversion rate was calculated as follows: negative conversion rate = (Number of rats showing bacterial translocation / Total number of rats in each group) × 100%.

**Vaginal secretions and cleanliness examination:** the condition of vaginal secretions (vaginal smears) of rats was observed on D0 and on D5/D10/D14 after drug administration, and photographs were taken.

**Vaginal pH testing:** vaginal pH testing is performed once a week.

**Vaginal lavage fluid colony counting:** after the last administration, vaginal lavage fluid from each group of rats was taken using sterile cotton swabs (strict quantitative measurement during operation), applied to blood agar medium (aseptic operation), and placed in an incubator at 37°C. Colony count was performed after 24 hours.

**Vaginal histopathological scoring:** vaginal tissue was taken for HE staining. The degree of inflammation was scored based on pathological changes including the smoothness of the vaginal mucosal tissue, congestion, edema, and inflammatory cell infiltration in the submucosa, and the muscle layer. 1. Degree of degeneration and necrosis of vaginal mucosal epithelial cell: normal and intact (score 0); mild (score "1"); moderate (score "2"); severe (score "3"); extremely severe (score "4"). 2. Degree of inflammatory cell infiltration (under each high-power microscope field of view): none (score "0"); very few, <25 (score "1"); mild, 26-50 (score "2"); moderate, 51-100 (score "3"); severe, >100 (score "4"). 3. Degree of vascular congestion and edema: none (score "0"); very few (score "1"); mild (score "2"); moderate (score "3"); severe (score "4"). The scores for each animal were added up to obtain the total score.

### Experimental results

### Effects of L-theanine aqueous solution on vaginal pH value in a rat model of mixed vaginitis

As shown in Table 2, compared with the normal control group, the vaginal pH values of rats in the model control group were significantly increased before administration and 1 week after administration (*P*≤0.01). Compared with the model control group, the vaginal pH values of rats in the 0.1w/w%, 0.5w/w%, and 2.5w/w% L-theanine aqueous solution groups and the compound metronidazole vaginal suppository group were significantly decreased 1 week after administration (*P*≤0.01). Compared with the compound metronidazole vaginal suppository group, the vaginal pH values of the 0.5w/w% and 2.5w/w% L-theanine aqueous solution groups were significantly decreased 1 week after administration (*P*≤0.01).

**Table 2. Effect of L-theanine aqueous solution on vaginal pH value (x̅ ± s, n=10)**

| Groups | Dosage (mL/rat) | Vaginal pH values | | |
|---|---|---|---|---|
| | | Before administration | Administration for 1w | Administration for 2w |
| Normal control group | 0.1 | **7.8±0.5** | **7.8±0.4** | **7.6±0.6** |
| Model control group | 0.1 | **10.3±0.2⁺⁺** | **9.8±0.4⁺⁺** | **9.4±0.2⁺⁺** |
| Compound metronidazole vaginal suppository group | 0.1g/rat | **10.1±0.2⁺⁺** | **8.2±0.6^{**}** | **8.2±0.2^{**}** |
| 0.1 w/w % L-theanine aqueous solution group | 0.1 | **10.1±0.4⁺⁺** | **7.8±0.5^{**}** | **7.4±0.4^{**▲▲}** |
| 0.5 w/w % L-theanine aqueous solution group | 0.1 | **10.1±0.4⁺⁺** | **7.2±0.4^{**▲▲}** | **7.2±0.2^{**▲▲}** |
| 2.5 w/w % L-theanine aqueous solution group | 0.1 | **10.0±0.5⁺⁺** | **7.3±0.5^{**▲▲}** | **7.3±0.3^{**▲▲}** |

| | | | | |
|---|---|---|---|---|
| Note: compared with the normal control group, ⁺⁺*P*≤0.01; compared with the model control group, ^{**}*P*≤0.01; compared with the compound metronidazole vaginal suppository group, ^{▲▲} *P*≤0.01. | | | | |

### Effect of L-theanine aqueous solution on vaginal bacterial colonization and cleanliness in a rat model of mixed vaginitis

As shown in Table 3, compared with the normal control group, the number of vaginal bacteria colonization in the model control group of rats was significantly increased before administration (*P*≤0.01). Compared with the model control group, the number of vaginal bacteria colonization in the compound metronidazole vaginal suppository group of rats was significantly decreased on D14 of administration (*P*≤0.05). The negative conversion rates of the model control group, compound metronidazole vaginal suppository group, 0.1 w/w% L-theanine aqueous solution group, 0.5 w/w% L-theanine aqueous solution group, and 2.5 w/w% L-theanine aqueous solution group were 30%, 40%, 20%, 20%, and 20%, respectively.

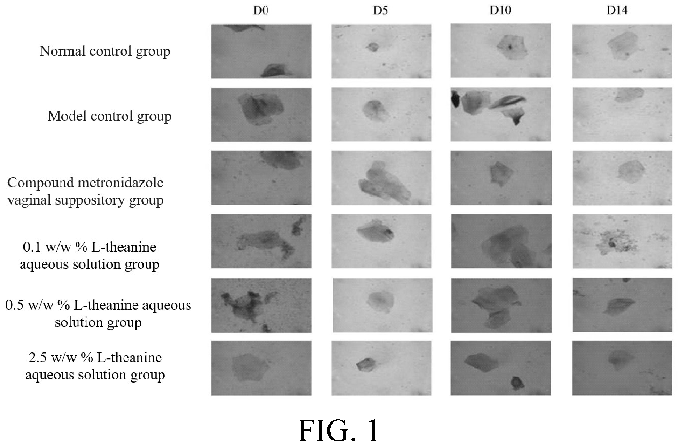

As shown in Table 4 and Figure 1, compared with the normal control group, the numbers of Gram bacteria colonization in the vagina of rats in the model control group were significantly increased on D0 and D5 of administration (*P*≤0.01). Compared with the model control group, the number of Gram bacteria colonization in the vagina of rats in the 0.1 w/w% L-theanine aqueous solution group was significantly decreased on D5 after administration (*P*≤0.05), the numbers of Gram bacteria colonization in the vagina of rats in the 2.5 w/w% L-theanine aqueous solution group were significantly decreased on D10 and D14 after administration (*P*≤0.05 or *P*≤0.01), and the numbers of Gram bacteria colonization in the vagina of rats in the compound metronidazole vaginal suppository group were significantly decreased on D5, D10, and D14 after administration (*P*≤0.05). Compared with the compound metronidazole vaginal suppository group, the numbers of Gram-bacteria colonization in the vagina of rats were significantly increased in the 0.1 w/w % L-theanine aqueous solution group on D14 after administration, in the 0.5 w/w% L-theanine aqueous solution group on D10 after administration, and in the 2.5 w/w% L-theanine aqueous solution group on D5 after administration (*P*≤0.05).

As shown in Table 5 and Figure 1, compared with the normal control group, the vaginal cleanliness grades of rats in the model control group were significantly increased from D0 to D14 after administration (*P*≤0.01). Compared with the model control group, the vaginal cleanliness grades of rats in the 0.1 w/w%, 0.5 w/w%, and 2.5 w/w% L-theanine aqueous solution groups were significantly decreased from D5 to D14 after administration (*P*≤0.05 or *P*≤0.01). Compared with the compound metronidazole vaginal suppository group, the vaginal cleanliness grades of rats in the 2.5 w/w% L-theanine aqueous solution group were significantly increased from D10 to D14 after administration, and the vaginal cleanliness grades of rats in the 0.1 w/w% and 0.5 w/w% L-theanine aqueous solution groups were significantly increased on D14 after administration (*P*≤0.05). Lactobacillus isolation and culture results showed that the increased colonies in the L-theanine aqueous solution group were mainly Lactobacillus.

**Table 3. Effect of L-theanine aqueous solution on vaginal bacterial colonization (x̅ ± s, n=10)**

| Groups | Dosage (mL/rat) | Vaginal bacterial colonization (numbers) | | |
|---|---|---|---|---|
| | | Before administration | D14 after Administration | Negative conversion rates (%) |
| Normal control group | 0.1 | 369.5±145.3 | 67.8±75.0 | -- |
| Model control group | 0.1 | **579.9**±**161.3**⁺⁺ | **150.3±127.3⁺⁺** | 30 |
| Compound metronidazole vaginal suppository group | 0.1g/rat | **579.8±172.6** | **113.2±128.7^{*}** | 40 |
| 0.1 w/w % L-theanine aqueous solution group | 0.1 | 579.2±175.1 | 137.4±131.1 | 20 |
| 0.5 w/w % L-theanine aqueous solution group | 0.1 | 588.4±182.7 | 123.3±98.0 | 30 |
| 2.5 w/w % L-theanine aqueous solution group | 0.1 | 577.1±141.2 | 126.9±146.7 | 30 |

| | | | | |
|---|---|---|---|---|
| Note: compared with the normal control group, ⁺⁺ *P*≤0.01; compared with the model control group, * *P*≤0.05. | | | | |

**Table 4. Effect of L-theanine aqueous solution on vaginal Gram bacteria (x̅ ± s, n=10)**

| Groups | Dosage (mL/rat) | Vaginal Gram bacteria (numbers) | | | |
|---|---|---|---|---|---|
| | | D0 before administration | D5 before administration | D10 before administration | D14 after administration |
| Normal control group | 0.1 | 1.3±2.5 | 1.3±1.7 | 17.8±31.6 | 19.3±21.9 |
| Model control group | 0.1 | **824.8±529.7⁺⁺** | **14.1±10.4**^{**+**+} | 17.0±22.5 | 9.7±7.1 |
| Compound metronidazole vaginal suppository group | 0.1g/rat | 665.9±432.4 | **5.9±5.2^{*}** | **4.4±4.4^{*}** | **2.2±2.4^{*}** |
| 0.1 w/w % L-theanine aqueous solution group | 0.1 | 701.1±495.4 | **5.0±7.8^{*}** | 17.8±26 | **24.4±32.7^{▲}** |
| 0.5 w/w % L-theanine aqueous solution group | 0.1 | 687.8±626.9 | 9.6±13.0 | **35.9±41.3^{▲}** | 5.4±4.7 |
| 2.5 w/w % L-theanine aqueous solution group | 0.1 | 904.1±467.2 | **22.9**±**34.1^{▲}** | **2.7±2.6^{*}** | **1.8±2.4^{**}** |

| | | | | | |
|---|---|---|---|---|---|
| Note: compared with the normal control group, ⁺⁺*P*≤0.01; compared with the model control group, ^{*}*P*≤0.05, *^{**}P≤*0.01; compared with the compound metronidazole vaginal suppository group, *^{▲}P≤*0.05*.* | | | | | |

**Table 5. Effect of L-theanine aqueous solution on vaginal cleanliness (x̅ ± s, n=10)**

| Groups | Dosage (mL/rat) | Cleanliness (grades) | | | |
|---|---|---|---|---|---|
| | | D0 before administration | D5 before administration | D10 before administration | D14 after administration |
| Normal control group | 0.1 | 1.47±0.34 | 1.18±0.15 | 1.24±0.13 | 1.24±0.13 |
| Model control group | 0.1 | **3.50**±**0.21⁺⁺** | **2.84±0.28⁺⁺** | 2.13**±0**.31⁺⁺ | **1.68±0.23⁺⁺** |
| Compound metronidazole vaginal suppository group | 0.1g/rat | 3.37±0.38 | **1.85±0.16^{**}** | 1.40**±**0.28^{**} | **1.18±0.15^{**}** |
| 0.1 w/w % L-theanine aqueous solution group | 0.1 | 3.58±0.29 | **1.88±0.15^{**}** | **1.58±0.29^{**}** | **1.35±0.2^{*▲}** |
| 0.5 w/w % L-theanine aqueous solution group | 0.1 | 3.71±0.26 | **1.84±0.24⁺⁺** | 1.67**±**0.29⁺⁺ | **1.34±0.13^{*▲}** |
| 2.5 w/w % L-theanine aqueous solution group | 0.1 | 3.62±0.27 | **1.73±0.32^{**}** | **1.68±0.32^{**▲}** | **1.37±0.31^{*▲}** |

| | | | | | |
|---|---|---|---|---|---|
| Note: compared with the normal control group, ⁺⁺ *P*≤0.01; compared with the model control group, ^{*}*P*≤0.05, ^{**}*P*≤0.01; compared with the compound metronidazole vaginal suppository group, ^{▲} *P*≤0.05. | | | | | |

### Effect of L-theanine aqueous solution on vaginal pathological changes in a rat model of mixed vaginitis

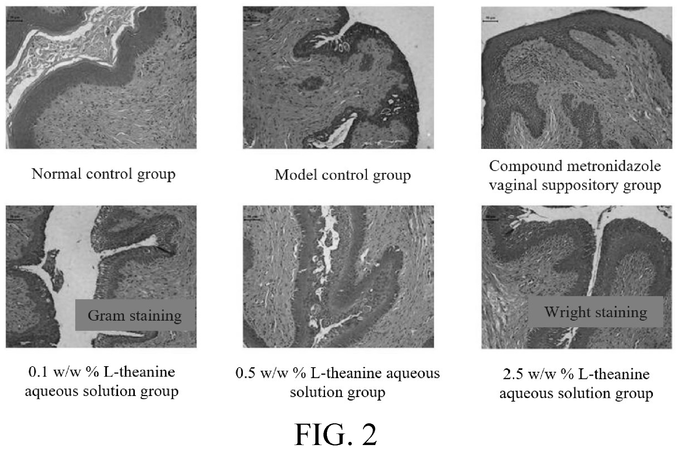

As shown in Table 6 and Figure 2, compared with the normal control group, the vaginal epithelial cell degeneration and necrosis, inflammatory cell infiltration, and total pathological score of rats in the model control group were significantly increased (*P*≤0.01). Compared with the model control group, the vaginal epithelial cell degeneration and necrosis, inflammatory cell infiltration, and total pathological score of the 0.1 w/w%, 0.5 w/w%, and 2.5 w/w% L-theanine aqueous solution groups and the compound metronidazole vaginal suppository group were significantly decreased (*P*≤0.05 or *P*≤0.01). Compared with the compound metronidazole vaginal suppository group, no significant differences were observed among the test substance groups.

**Table 6. Effect of L-theanine aqueous solution on vaginal pathological scores (x̅ ± s, n=10)**

| Groups | Dosage (mL/rat) | Pathological scores (score) | | | | |
|---|---|---|---|---|---|---|
| | | Epithelial cell degeneration and necrosis | Inflammatory cell infiltration | Vascular congestion | Edema | Total score |
| Normal control group | 0.1 | 0.0±0.0 | 0.0±0.0 | 0.0±0.0 | 0.0±0.0 | 0.0±0.0 |
| Model control group | 0.1 | **2.2±1.**5⁺⁺ | **1.9±0.9⁺⁺** | 0.0±0.0 | 0.0±0.0 | **4.1±2.2⁺⁺** |
| Compound metronidazole vaginal suppository group | 0.1g/rat | **1.3±0.8^{*}** | **1.0±1.5^{*}** | 0.0±0.0 | 0.0±0.0 | **2.3±1.7^{**}** |
| 0.1 w/w % L-theanine aqueous solution group | 0.1 | **0.8±0.9^{**}** | **0.4±1.0^{**}** | 0.0±0.0 | 0.0±0.0 | **1.2±1.2^{**}** |
| 0.5 w/w % L-theanine aqueous solution group | 0.1 | **0.6±0.7^{**}** | **0.6±1.1^{**}** | 0.0±0.0 | 0.0±0.0 | **1.2±1.3^{**}** |
| 2.5 w/w % L-theanine aqueous solution group | 0.1 | **1.4±0.8^{*}** | **0.2±0.4^{**}** | 0.0±0.0 | 0.0±0.0 | **1.6±0.8^{**}** |

| | | | | | | |
|---|---|---|---|---|---|---|
| Note: compared with the normal control group, ⁺⁺ *P*≤0.01; compared with the model control group, ^{*}*P≤*0.05, ^{**}*P*≤0.01. | | | | | | |

As can be seen from Figures 1 and 2, and Tables 1, 2, 3, 4, 5, and 6, L-theanine aqueous solution has a significant therapeutic effect on a rat model of mixed vaginitis.

It should be declared that the above-described invention content and specific embodiments are intended to demonstrate the practical application of the technical solution provided by the present invention and should not be construed as limiting the scope of protection of the present invention. Those skilled in the art can make various modifications, equivalent substitutions, or improvements within the spirit and principles of the present invention.

## Claims

1. Use of L-theanine in the preparation of a formulation for treating vaginitis.

2. The use according to claim 1, wherein the vaginitis comprises bacterial vaginosis, aerobic vaginitis, fungal vaginitis, atrophic vaginitis, and mixed vaginitis.

3. The use according to claim 1, wherein the administration route of L-theanine is vaginal administration, and the effective therapeutic dosage of L-theanine for vaginal administration is 10 mg ~ 250 mg per day.

4. The use according to claim 3, wherein the effective therapeutic concentration of L-theanine for vaginal administration is 0.1 w/w% ~ 2.5 w/w%.
